# EUROPEAN PATENT APPLICATION

(11) **EP 4 481 102 A1**
(43) Date of publication of application: **25.12.2024**
(21) Application number: 23756644.3
(22) Date of filing: 16.02.2023
(51) Int. Cl.: D06F 58/20, D06F 58/10, D06F 71/34, D06F 71/36, D06F 71/29, D06F 73/02, A61L 2/07

(54) **CLOTHING PROCESSING APPARATUS**

(30) Priority: 18.02.2022 KR 20220021396
(71) Applicant: LG Electronics Inc., Yeongdeungpo-gu Seoul 07336 (KR)
(72) Inventor: SHIN, Woosup, Seoul 08592 (KR); SONG, Sungho, Seoul 08592 (KR); YE, Sungmin, Seoul 08592 (KR)
(74) Representative: Vossius & Partner Patentanwälte Rechtsanwälte mbB
(86) International application number: PCT/KR2023/002254
(87) International publication number: WO 2023/158232

(57) **Abstract**

The present disclosure relates to a clothing processing apparatus comprising: a cabinet which has an opening provided at the front thereof; an inner case which is provided in the cabinet and in which clothes are accommodated; a machine room which is provided at the bottom of the inner case and generates hot air or steam into the inner case; a door which is pivotally provided at the cabinet; a pressing part which presses the clothes; an air moving part which supplies at least one of the hot air and the steam; an air inlet part; an air outlet part; and a duct part.

## Description

### TECHNICAL FIELD

The present disclosure relates to a clothing processing apparatus, and more particularly, to a clothing processing apparatus for removing wrinkles from laundry and deodorizing the laundry by supplying hot air and steam to the laundry.

### BACKGROUND

The clothing processing apparatus refers to an apparatus developed for washing and drying laundry and removing wrinkles at home and in laundries. A concept of the clothing processing apparatus includes a washing machine that washes laundry, a dryer that dries laundry, a washing/drying machine that has both washing and drying functions, a laundry management machine that refreshes laundry, and a steamer that removes wrinkles from laundry.

Recently, a clothing processing apparatus has appeared to keep laundry comfortable and clean without having to soak the laundry in water and wash the laundry with detergent.

The conventional clothing processing apparatus may remove fine dust attached to laundry, remove odors, dry laundry, and add fragrance to the laundry.

In addition, the conventional clothing processing apparatus may prevent static electricity from being generated, remove wrinkles from laundry by using dehumidified air or steam, and sterilize and disinfect laundry.

A process of treating laundry as such is defined below as refreshing.

FIG. 1 shows a conventional clothing processing apparatus. (Refer to Korean Patent Application No. 10-2020-0116692)

The conventional clothing processing apparatus includes a cabinet 100 having an opening at a front side, an inner case 200 providing a space for holding laundry inside the cabinet 100, a machine room 700 supplying hot air to the inner case 200, and a door 300 rotatably provided on the cabinet 100 to open and close the cabinet 100.

The inner case 200 may include a holder 400 on which laundry is to be held. Accordingly, the laundry may be held on the holder 400 together with a hanger and refreshed by hot air or steam supplied from the machine room 700.

In the conventional clothing processing apparatus, a pressurizer 500 is separately provided in the door 300 and a hanger unit 511 on which laundry such as pants is to be held may be provided.

The pressurizer 500 may pressurize the laundry to remove wrinkles generated in the laundry and form wrinkles in specific areas of the laundry.

However, the pressurizer 500 needs to maintain a pressurized state of the laundry to remove wrinkles from the laundry held on the door 300. Accordingly, there is a limitation that laundry pressurized by the pressurizer 500 may not be exposed to hot air or steam supplied from the machine room due to the pressurizer 500. As a result, paradoxically, the laundry pressurized by the pressurizer 500 not only does not refresh properly, but also has a problem in that wrinkles may not be removed properly.

To resolve this, a clothing processing apparatus has appeared to add a device for separately supplying hot air or steam to the laundry to the pressurizer 500. (Refer to Korean Patent Application No. 10-2021-0098406)

However, the conventional clothing processing apparatus also requires that not only a device for supplying hot air or steam, but also an entire path through which hot air or steam passes be additionally installed in a door 500. In this case, there is a problem that a structure of the door 500 becomes very complicated.

As in the case of a conventional clothing processing apparatus, when a device for supplying hot air or steam is added to the door 500 in addition to the machine room, the device installed in the machine room and on the door 500 needs to be controlled or managed simultaneously, which causes the complexity of the device.

In particular, when a separate heater is additionally installed in the door 500 in addition to the machine room as in the conventional clothing processing apparatus, there is a fundamental problem that energy consumption becomes extreme and an allowable current value is exceeded when the machine room and the heater are operated simultaneously.

Accordingly, the conventional clothing processing apparatus needs to alternately drive the heater provided on a machine room 300 and the door 500, and as a result, the machine room may not supply sufficient hot air and steam to the inside of the inner case, which causes a problem in that an effect of treating laundry held on the holder 400 is drastically reduced.

### DISCLOSURE

### Technical Problem

The present disclosure provides a clothing processing apparatus for supplying one or more of hot air and steam generated in a machine room to a pressurizer installed in a door to pressurize laundry.

The present disclosure provides a clothing processing apparatus for supplying both hot air or steam generated in a machine room to laundry held on an inner case and the laundry pressurized by the door.

The present disclosure provides a clothing processing apparatus for supplying steam and hot air supplied into an inner case on which laundry is held, up to laundry pressurized by the pressurizer.

The present disclosure provides a clothing processing apparatus for discharging hot air or steam supplied to an inner case to the outside of a cabinet or circulating the hot air or the steam to a machine room again.

The present disclosure provides a clothing processing apparatus for smoothly moving at least one of hot air and steam to a duct installed in a door.

### Technical Solution

To achieve the above objects, a clothing processing apparatus according to the present disclosure may include a pressurizer configured to pressurize laundry on a door and a duct unit to which hot and steam to be supplied to the pressurizer move.

The duct unit may include an exhaust unit configured to supply hot air and steam to the pressurizer and an intake unit configured to receive the hot air and the steam.

The exhaust unit may be located to at least partially overlap the pressurizer.

The exhaust unit may be provided with an area equal to or larger than that of the pressurizer, and thus may evenly supply at least one of hot air or steam to the entire pressurizer.

The pressurizer may include a plurality of through holes that allow the hot air and the steam to pass through without obstructing flow of the hot air and the steam.

The intake unit may be provided with a less area than the exhaust unit.

The intake unit may be located below the exhaust unit and located closer to a lower end of the door or a lower end of the duct unit than to an upper end of the door or an upper end of the duct unit.

The intake unit may be located below the pressurizer.

The intake unit may include a fan that moves hot air or steam. Accordingly, the intake unit may be located not to face the hanging unit or a holder that holds the laundry. This is to prevent the laundry from being sucked into the fan or from blocking the intake unit.

It may be advantageous to have the fan located closer to a lower portion of the door, thereby preventing vibration and noise from radiating outside the cabinet.

The intake unit may be located not to face the through hole through which hot air and steam are discharged or sucked in the machine room. As a result, the intake unit may be prevented from blocking a flow path of hot air and steam flowing from the machine room to the inner case or being discharged from the machine room to the inner case.

To this end, the intake unit may be located in parallel to the exhaust unit.

The duct unit may further include an exhaust duct facing the pressurizer and having the exhaust unit, and an intake duct extending downward from the exhaust duct and including the intake unit. The intake duct may further protrude into the inner case compared with the exhaust duct.

The intake duct may be located above the machine room.

The fan may include an impeller configured to move the hot air or the steam to the exhaust duct from an inside of the intake duct, and a driver including a rotation shaft that rotates the impeller, and a direction of the rotation shaft may be located in parallel to a thickness direction of the door to prevent the intake duct from excessively protruding.

The air moving unit may include a guide located between the intake unit and the door and configured to guide at least one of the hot air and the steam introduced from the intake unit to the exhaust duct.

The guide may include one or more vanes located between the fan and the door

To achieve the above objects, a clothing processing apparatus according to the present disclosure may include a duct unit that passes one or more of hot air and steam through the door opening and closing the cabinet or moves one or more of hot air and steam to the door. The duct unit may include a fan installed thereon.

At least a portion of the fan may be installed inside the duct unit.

The duct unit may include an inlet into which at least one of air inside the inner case or hot air and steam supplied from the machine room is introduced.

The door may include an external discharge unit configured to guide at least one of the air, the hot air, or the steam introduced into the inlet to an outside of the cabinet, and an external damper configured to open and close the external discharge unit.

The door may include an internal circulation unit configured to re-guide at least one of the air, the hot air, or the steam introduced into the inlet to the machine room, and an internal damper configured to open and close the internal circulation unit.

To achieve the above objects, a clothing processing apparatus according to the present disclosure may include a duct unit providing a path through which at least one of the hot air and the steam moves, an intake unit into which at least one of the hot air or the steam is introduced to pass through the duct unit, and an exhaust unit spaced apart from the intake unit and configured to discharge at least one of the hot air or the steam introduced into the intake unit toward the laundry

The duct unit may be located in a length direction of the door and may be located above the machine room.

The exhaust unit may be provided to pass through one surface of the duct unit, which faces an inside of the inner case.

The intake unit may be provided to pass through one surface of the duct unit, which faces an inside of the inner case.

The clothing processing apparatus may further include a fan provided inside the duct unit and configured to move one or more of the hot air and the steam to the exhaust unit from the intake unit.

### Advantageous Effects

The present disclosure has an effect of supplying one or more of hot air and steam generated in a machine room to a pressurizer installed in a door to pressurize laundry.

The present disclosure has an effect of supplying both hot air or steam generated in a machine room to laundry held on an inner case and the laundry pressurized by the door.

The present disclosure has an effect of supplying steam and hot air supplied into an inner case on which laundry is held, up to laundry pressurized by the pressurizer.

The present disclosure has an effect of discharging hot air or steam supplied to an inner case to the outside of a cabinet or circulating the hot air or the steam to a machine room again.

The present disclosure has an effect of smoothly moving at least one of hot air and steam to a duct installed in a door.

The present disclosure has an effect in which laundry pressurized by a pressurizer is sufficiently exposed to one or more of hot air and steam.

### BRIEF DESCRIPTION OF THE DRAWINGS

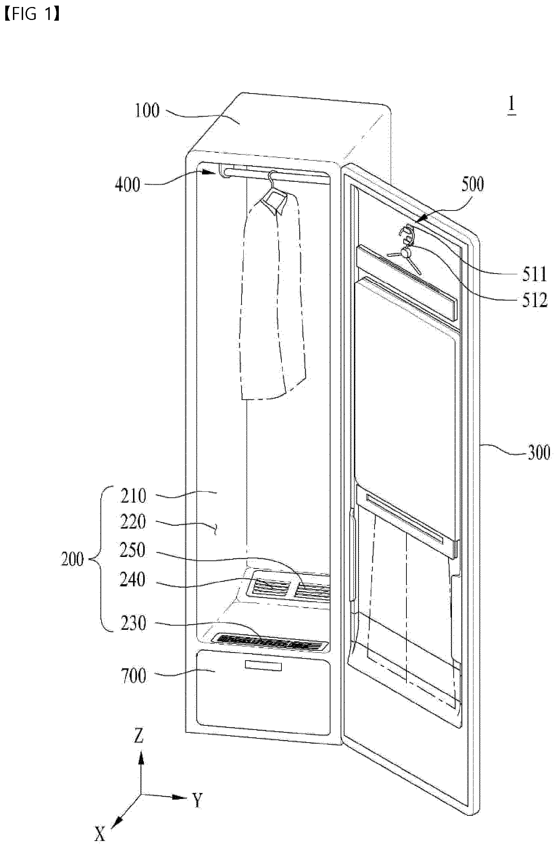

FIG. 1 illustrates a conventional clothing processing apparatus.
FIG. 2 illustrates a structure of a clothing processing apparatus according to the present disclosure.
FIG. 3 illustrates a machine room according to an embodiment.
FIG. 4 illustrates a door of a clothing processing apparatus and a coupling structure of the door, according to the present disclosure.
FIG. 5 illustrates a pressurizer coupled to a door, according to an embodiment.
FIG. 6 illustrates a structure providing elasticity of a pressurizer.
FIG. 7 illustrates a cross-sectional structure in a width direction of a door.
FIG. 8 illustrates a cross-sectional structure in a length direction of a door.
FIG. 9 is a positional relationship between a machine room and a duct unit.
FIG. 10 illustrates a structure of a fan installed on a door.
FIG. 11 illustrates an air moving direction of a duct unit installed in a door.
FIG. 12 illustrates a clothing processing apparatus according to a further embodiment of the present disclosure.

### DETAILED DESCRIPTION

Hereinafter, embodiments disclosed in this specification will be described in detail with reference to the attached drawings. In this specification, identical or similar components in different embodiments are given identical or similar reference numbers, and their descriptions are replaced with the first description. As used herein, singular expressions include plural expressions unless the context clearly indicates otherwise. In the description of the present disclosure, certain detailed explanations of related art are omitted when it is deemed that they may unnecessarily obscure the essence of the present disclosure. The features of the present disclosure will be more clearly understood from the accompanying drawings and should not be limited by the accompanying drawings.

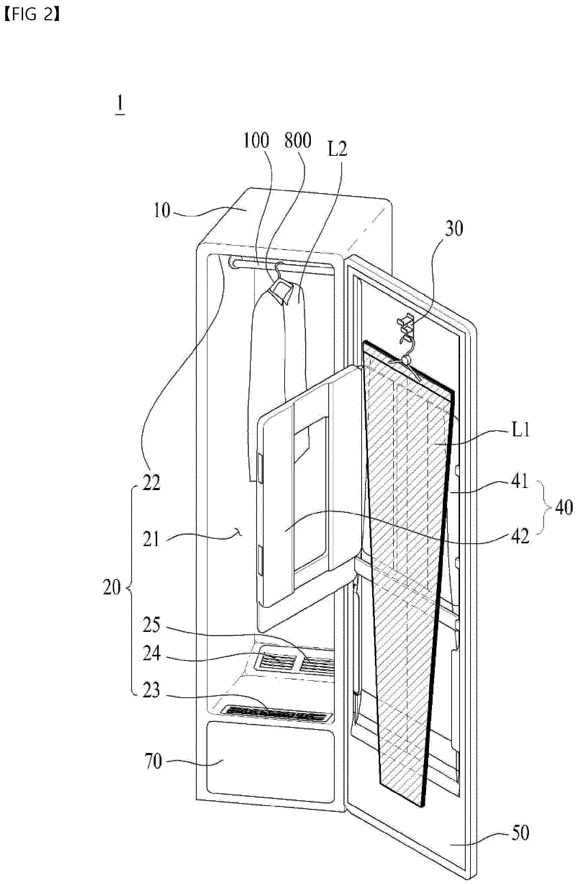

FIG. 2 illustrates a structure of a clothing processing apparatus according to the present disclosure.

The clothing processing apparatus according to the present disclosure may include a cabinet 10 having an opening at a front side, a door 50 rotatably provided on the cabinet 10 to open and close the opening, an inner case 20 provided on the cabinet to provide an accommodation space 21 that accommodates laundry L therein, and a machine room 70 connected to the inner case 20 and provided to generate at least one of air (hot air) and moisture supplied to the accommodation space 21.

The inner case 20 may include a hanging unit 100 on which the laundry is to be held in the accommodation space 21. The laundry held on the hanging unit 100 may mainly correspond to an upper garment L2.

The cabinet 10 may define an outer appearance of the clothing processing apparatus and may be provided with a height greater than a width. The cabinet 10 may store laundry such as a lower garment with a length greater than a width, in an unfolded state. Accordingly, the cabinet 10 may prevent wrinkles from being generated when a laundry L2 is stored in the inner case 20.

The cabinet 10 may include metal, but may also include resin material such as reinforced plastic as long as strength is to be maintained.

The inner case 20 may be accommodated inside the cabinet 10 to form a space for accommodating the laundry. The inner case 20 may be provided with a height greater than a width, like the cabinet 10, and may be provided in a case shape with an opening at a front side.

The machine room 70 may be provided at a lower side of the cabinet 10, and thus the height of the inner case 20 may be provided to be less than a height of the cabinet 10.

The inner case 20 may include a material for maintaining strength without being deformed by hot air or steam supplied from the machine room 70. The inner case 20 may include a material that does not chemically react with foreign substances separated from the laundry during a process of exposing the laundry to the hot air or steam. For example, the inner case 20 may include a styrene resin series such as ABS or ASA.

The machine room 70 may be provided separated and partitioned from the inner case 20 within the cabinet 10.

The machine room 70 may be provided below the inner case 20 to supply hot air or steam with a lower density than air to the accommodation space 21.

The inner case 20 and the machine room 70 may be provided to communicate with each other.

To this end, the inner case 20 may be connected to the machine room 70 and may include a plurality of through holes 23, 24, and 25 through which one or more of hot air or steam may pass.

Accordingly, the inner case 20 may be supplied with hot air or moisture from the machine room 70, or the hot air or steam supplied to the machine room 70 may be discharged again together with foreign substances discharged from laundry.

The plurality of through holes may include a discharge hole 25 through which air generated in the machine room 70 is discharged, and an intake port 23 through which air inside the inner case 20 is sucked into the machine room 70.

The discharge hole 25 may be located at a rear side of a lower surface of the inner case 20, and the intake port 23 may be located at a front side of the lower surface of the inner case 20.

The discharge hole 25 may be located to be inclined upward from the lower surface of the inner case 20 to directly supply hot air to laundry held on the hanging unit 100.

When a steam generator that generates steam is provided in the machine room 70, the plurality of through holes may further include a moisture port 24 through which moisture that separates foreign substances adsorbed on laundry is discharged. The moisture port 24 may be located at one side of the discharge hole 25 and may be located parallel to the discharge hole 25.

The air supplied from the machine room 70 to the accommodation space 21 may generally be air (hot air) heated by a heat pump or a heater. Moisture supplied from the machine room 70 to the accommodation space 21 may be steam generated by boiling water.

The hot air or steam has a lighter density than air, and thus when the machine room 70 is located below the inner case 20 and the machine room 70 and the inner case 20 are connected through the plurality of through holes 23, 24, and 25, hot air and steam may be normally supplied inside the accommodation space 21 even without a separate blower. The internal configuration of the above machine room 70 is described below.

The door 50 may be rotatably coupled to the cabinet 10.

The door 50 may be provided longer than the inner case 20 and may be provided with a length corresponding to the cabinet 10.

The door 50 may be provided to shield not only the opening of the inner case 20 but also a front side of the machine room 70. Accordingly, the door 50 may prevent hot air or moisture supplied to the accommodation space 21 from leaking out to the outside and block heat generated in the machine room 70 from being transferred to the outside.

The door 50 is provided to open and close the front side of the inner case 20, and thus inner surfaces of the inner case 20 and inner surfaces of the door 50 may form a sealed accommodation space 21.

The hanging unit 100 may be provided inside the inner case 20 to hold laundry in the accommodation space 21. The hanging unit 100 may be located at an upper portion of the inner case 20 such that the laundry L may be held in the accommodation space 21 in an unfolded state without being folded.

The hanging unit 100 may be coupled to an upper portion of a lateral surface of the inner case 20 and may also be coupled to the upper surface of the inner case 20.

The hanging unit 100 may be provided in a shape in which the laundry itself is held, or may be provided in such a way that a hanger holding the laundry is hung.

The hanging unit 100 may extend in a left-right direction or width direction of the inner case 20 to hold multiple laundries.

The laundry held on the hanging unit 100 may be evenly exposed to hot air and moisture supplied from the machine room 70 by floating inside the accommodation space 21.

The hanging unit 100 may be provided to be swayed by a separate driver provided between the inner case 20 and the cabinet 10. As a result, dust or foreign substances attached to the laundry may be easily separated.

The door 50 may further include a holder 30 on which laundry is to be additionally held.

The holder 30 may be coupled to an inner surface of the door 20 to hold the laundry or may be provided to hold a hanger to which the laundry is fixed.

The holder 30 may be located offset from the upper portion of the door 50 so that laundry held on the door 50 is not wrinkled. For example, the holder 30 may be located at a height corresponding to the height of the hanging unit 100.

The door 50 may be provided with a height greater than a width, and thus laundry held on the door 50 may mainly correspond to lower garment L 1.

The laundry held on the door 50 is held separately from the hanging unit 100 and is located in a front side of the accommodation space 21. Therefore, laundry accommodating capacity of the clothing processing apparatus may be further improved due to the holder 30.

The clothing processing apparatus according to the present disclosure may further include a pressurizer 40 that is coupled to the door 50 and may pressurize laundry held on the holder 30.

When the laundry is held by the holder 30 and located in an unfolded state, the pressurizer 40 may be provided to pressurize one or both surfaces of the laundry. Accordingly, the pressurizer 40 may remove wrinkles from laundry or form intentional creases.

Laundry held on the door 50 may be exposed to one or more of hot air or steam supplied to the inner case 20, and thus may be refreshed, and wrinkles may be removed by being pressurized by the pressurizer 40. In this case, refreshing may be a concept that includes one or more of deodorization, dehumidification, heating, wrinkle removal, and foreign substance separation.

The pressurizer 40 may include a support portion 41 that is fixed to the inner surface of the door 50 and supports one surface of laundry held on the holder 30, and a compression portion 42 that is rotatably coupled to the door 50 or the support portion 41 and presses the other surface of the laundry supported on the support portion 41.

The laundry may have wrinkles formed at both corners as wrinkles are removed by being pressed on both surfaces by the support portion 41 and the compression portion 42. A specific structure of the pressurizer 40 will be described below.

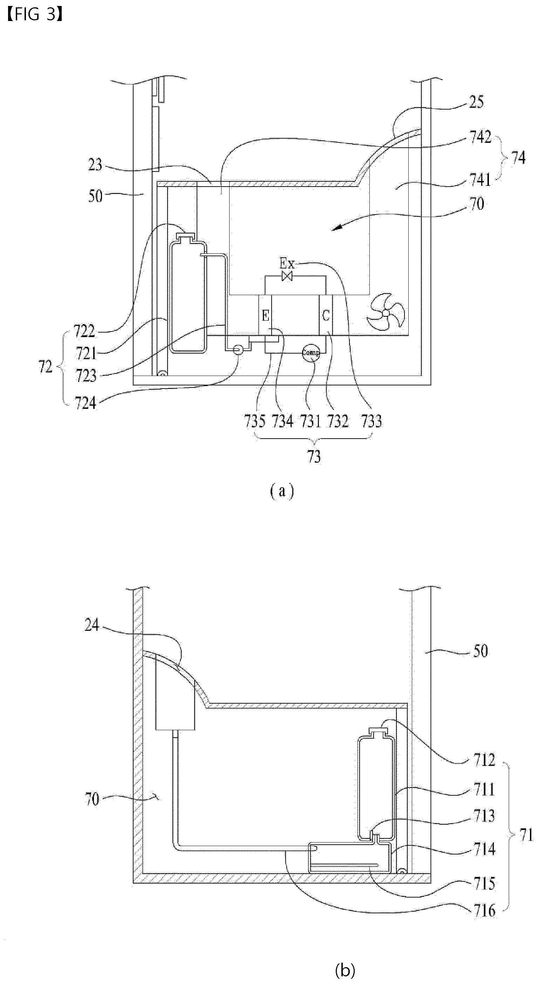

FIG. 3 illustrates an example of a structure of the machine room 70.

The machine room 70 may include at least one of a heater 73 that supplies air (heated air or unheated air) to the accommodation space 21 and a moisture supply unit 71 that supplies moisture (steam or mist) to the accommodation space 21.

Referring to FIG. 3(a), the heater 73 may include a circulation duct 74 that circulates air inside the accommodation space 21, a heat pump 73 that exchanges heat with air flowing along the circulation duct 74, and a blower 75 that provides power to allow air inside the accommodation space 21 to flow along the circulation duct 74.

The circulation duct 74 communicates with the inside of the accommodation space 21 through the discharge hole 25 and the intake port 23 provided in the accommodation space 21. One end of the circulation duct 74 may be connected to the intake port 23 and the other end may be connected to the discharge hole 25.

Accordingly, air inside the inner case 20 may pass through the circulation duct 74 and may be supplied back to the inner case 20.

A device for dehumidifying and heating the introduced air, for example, a heat pump may be installed inside the circulation duct 74.

The circulation duct 74 may include a discharge duct 741 that extends from one end to communicate with the discharge hole 25 and an intake duct 742 that extends from the other end to communicate with the intake port 23.

The heat pump may be installed between the discharge duct 741 and the intake duct 742 in the circulation duct 74 and supported on a bottom surface of the machine room 70.

The heat pump may include an evaporator 734 provided inside the circulation duct 74, a condenser 732 provided inside the circulation duct 74 separated from the evaporator 734, and a compressor 731 and an expander 733 that are located outside the circulation duct.

The evaporator 734, the compressor 731, the condenser 732, and the expander 733 may be connected to each other through a refrigerant pipe 735.

A pressure of a refrigerant that passes through the condenser 732 decreases and simultaneously a temperature of the refrigerant drops rapidly while the refrigerant passes through the expander 733.

Moisture in the air may be condensed into water while the cooled air passes through the evaporator 734 and air introduced into the intake duct 742 is cooled.

The refrigerant passing through the evaporator 734 is fed into the compressor 731 and compressed, thereby becoming a high temperature and high pressure state.

The refrigerant passing through the compressor 731 is fed into the condenser 732, and the condenser 732 generates hot air by reheating the low-temperature air passing through the evaporator 734. The refrigerant passing through the condenser 732 may be introduced back to the evaporator 734 while passing through the expander 733 again.

The blower 75 may be located inside the circulation duct 74 and may provide power to introduce air of the inner case 20 to the intake duct 742 and discharge the air to the discharge duct 741.

The machine room 70 may include a drain 72 that collects condensed water passing through the evaporator 734.

The drain 72 may include a drain tank 721 detachably installed in the machine room 70, a drain pipe 723 that supplies condensate inside the circulation duct 74 to the drain tank 721, and a drain pump 724 that collects the condensate collected at the lower portion of the evaporator into the drain tank 721.

The drain tank 721 may be provided in any shape as long as the drain tank 721 may provide a space in which liquid is to be stored. The drain tank 721 may include a drain plug 722 to discharge liquid stored inside the drain tank 721.

Referring to FIG. 3(b), the moisture supply unit 71 may include a storage unit 714 provided inside the machine room 70, a heater 715 provided inside the storage unit 714, and a moisture supply pipe 716 connecting the storage unit 714 to the moisture port 25.

The storage unit 714 is a device for storing water for generating steam, the heater 715 is a device for heating the water stored inside the storage unit 714 and converting the water into steam, and the moisture supply pipe 716 is a device for guiding the steam inside the storage unit 714 to the inner case 20.

Therefore, the heater 715 may be provided adjacent to the bottom surface of the storage unit 714, and the moisture supply pipe 716 may be located on the upper surface (or the uppermost portion) of the storage unit 714.

The storage unit 714 may receive water through a water supply unit, and the water supply unit may be provided as a water supply tank 711 that is detachably installed in the machine room 70.

In this case, the moisture supply unit 71 may further include a connector 713 that detachably connects the storage unit 714 and the water supply tank 714.

The water supply tank 714 may further include a water supply plug 712 to prevent water that generates the steam from leaking to the outside.

The water contained in the water supply tank 714 may pass through the connector 713 and is heated by a heater in the storage unit 714 and converted into steam, and the steam may pass through the moisture supply pipe 716 and may be discharged to the steam port 24. The moisture supply unit 71 may be provided independently without being connected to the circulation duct 74.

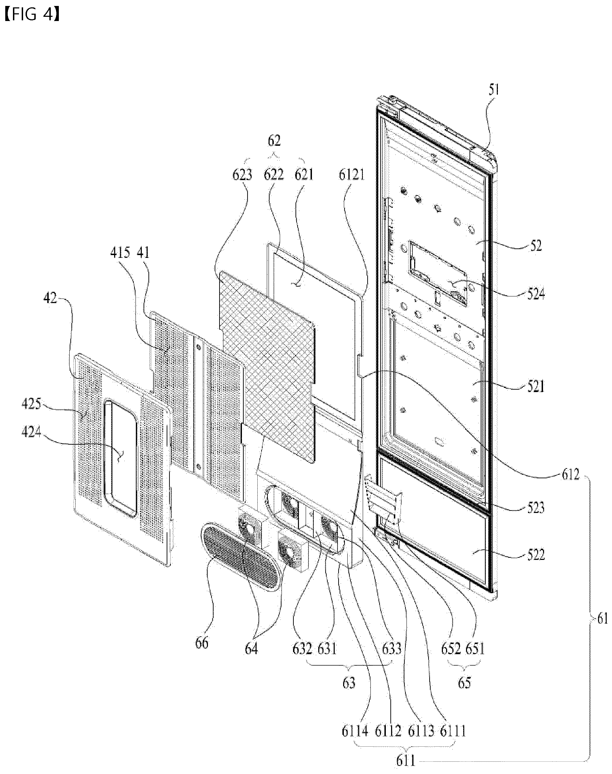

FIG. 4 illustrates the door 50 of a clothing processing apparatus according to an embodiment of the present disclosure.

The door 50 may be rotatably coupled to the cabinet 10 to shield the inner case 20 and may also shield the machine room 70 from being exposed to the outside.

The door 50 may include an outer surface 51 defining one surface of the cabinet 10 and an inner surface 52 defining the accommodation space 21 together with the inner case 20.

The door 50 may include a panel through hole 524 in which a control panel is installed.

The door 50 may include the holder 30 on which the laundry is to be held. The pressurizer 40 may be coupled to the door 50 and pressurize laundry held on the holder 30.

The pressurizer 40 is provided to pressurize both surfaces of the laundry. Accordingly, laundry pressurized by the pressurizer 40 may be difficult to expose to hot air and steam discharged from the machine room 70, and thus may not be refreshed or wrinkles may be difficult to remove.

To prevent this, the clothing processing apparatus according to the present disclosure may further include an air moving unit 60 provided in the door 50 to supply hot air and steam to laundry held on the pressurizer 40.

The air moving unit 60 may be provided to receive hot air or steam supplied inside the inner case 20 and supply the hot air or steam to the pressurizer 40. The air moving unit 60 may be provided to directly communicate with the machine room 70 to move hot air or steam supplied from the machine room 70 to the pressurizer 40.

As a result, the door 50 may refresh or remove wrinkles from both laundry held on the hanging unit 100 and laundry pressurized by the pressurizer 40 by using the hot air and steam generated in one machine room 70 without a need for a separate device for generating hot air or steam.

For example, the air moving unit 60 may include a duct unit 61 that is coupled to an inner surface 52 of the door 50 or is provided inside the door 50 to provide a space in which one or more of the hot air and steam moves, and an discharge unit 62 that supplies the hot air and steam moving in the duct unit 61 to the pressurizer 40.

The duct unit 61 may be provided in a shape of a duct or pipe that may extends along a height direction or length direction of the door 50.

The duct unit 61 may be provided to provide a path through which at least one of the hot air and steam moves in one direction or in the height direction.

The discharge unit 62 may be provided to pass through the duct unit 61 to at least partially overlap the pressurizer 40. Accordingly, at least one of the hot air and steam supplied from the discharge unit 62 may be directly supplied to the laundry pressurized in the pressurizer 40.

The pressurizer 40 may include the support portion 41 that faces the duct unit 61 or the discharge unit 62 and supports one surface of the laundry, and the compression portion 42 that is rotatably coupled to the door 50 or the support portion 41 and pressurizes the laundry toward the support.

In this case, at least one of the support portion 41 and the compression portion 42 may include a plurality of holes through which one or more of the hot air and steam discharged from the discharge unit 62 passes. Accordingly, one or more of the hot air and steam discharged from the discharge unit 62 may pass through the pressurizer 40 and may be supplied directly to the laundry.

The support portion 41 may fixedly face the discharge unit 62 to support one surface of the laundry and may include a plurality of support holes 415 through which the hot air and steam pass. The support portion 41 may be evenly distributed on a surface of the support portion 41.

Thus, the hot air and steam supplied from the discharge unit 62 may pass through the support hole 415 and be supplied to the laundry.

The compression portion 42 may be provided to face the support portion 41 and pressurize laundry supported by the support portion 41. The compression portion 42 may include a plurality of compression holes 425 through which the hot air and steam are capable of passing. The compression hole 425 may be located at a position corresponding to the support hole 415 and may be provided evenly distributed on the surface of the compression portion 42.

Accordingly, the steam and hot air passing through the support portion 41 may be smoothly discharged through the compression portion 42 into the accommodation space 21 of the inner case 20.

The compression portion 42 may further include a middle hole 424 that is formed to completely pass through the center such that the steam and hot air may be discharged more smoothly. The middle hole 424 may be not only provided to avoid a sewing line of the lower garment L1, but may also form a path through which steam and hot air pass. As a result, flow resistance applied to the hot air and steam discharged from the discharge unit 62 may be reduced, thereby inducing the hot air and steam to be continuously discharged from the discharge unit 62.

The duct unit 61 may further include an intake unit 63 provided to supply the hot air and steam. The intake unit 63 may be formed to pass through the duct unit 61 and may be spaced apart from the discharge unit 62.

The intake unit 63 may be spaced apart from the pressurizer 40. As such, it may be possible to prevent one or more of the hot air and steam discharged to the pressurizer 40 from being reabsorbed into the intake unit 63.

When intake unit 63 is located closer to an upper end of the door 50, the center of gravity of the duct unit 61 is located closer to the upper end of the door 50. Therefore, when vibration occurs in the duct unit 61 and the pressurizer 40, vibration and noise generated in the door 50 may be radiated more loudly to the outside of the cabinet 10.

In addition, when a fan 64 described below is installed in the intake unit 63, vibration or noise generated from the fan 64 may be radiated more loudly to the outside of the cabinet 10.

To ensure a space for installing the fan 64, when the intake unit 63 protrudes further into the inner case 20, there is a risk that the intake unit 63 may collide or interfere with the hanging unit 100 and the holder 30. Accordingly, the intake unit 63 may be located not to face the hanging unit 100 or the holder 30.

Considering this comprehensively, the intake unit 63 may be located closer to a lower end from among an upper end and the lower end of the door 50. The intake unit 63 may be located below the pressurizer 40 and below the discharge unit 62. Accordingly, the center of gravity of the entire duct unit 61 may be moved further to the lower end of the door 50, and thus the amplitude of vibration or noise generated from or transmitted to the door 50 may be reduced, and the size of noise and vibration radiated to the outside of the cabinet 10 may be further reduced.

The duct unit 61 may include an intake duct 611 including the intake unit 63 and an exhaust duct 612 including the discharge unit 62. The exhaust duct 612 may be provided to face the pressurizer 40, and the intake duct 611 may extend lower than the exhaust duct 612.

The exhaust duct 612 and the intake duct 611 need to ensure a path therein through which hot air and steam pass, and thus may protrude from the inner surface 52 of the door 50 toward the accommodation space 21. Even if the exhaust duct 612 and intake duct 611 are accommodated and provided inside the door 50, the inner surface 52 of the door 50 may protrude toward the accommodation space 21.

The exhaust duct 612 and the intake duct 611 may be located above machine room 70. Accordingly, it may be possible to prevent the intake duct 611 from colliding with the machine room 70 or the door 50 from becoming excessively thick to ensure a cross-sectional area of the intake duct 611.

The intake duct 611 may have a wider cross-sectional area than the exhaust duct 612. That is, the intake duct 611 may protrude toward the accommodation space 21 more significantly than the exhaust duct 612. Thus, this may induce more air to flow into the intake duct 611. The exhaust duct 612 may function as a diffuser to increase a flow rate of hot air and steam discharged to the pressurizer 40.

The condensed moisture or condensed steam from the laundry held on the holder 30 may be guided to a bottom surface of the inner case 20 while flowing along the protruding intake duct 611.

The intake duct 611 may include an extension surface 6111 extending downward from the exhaust duct 612 and extending toward the inside of the inner case 20, an installation surface 6112 extending from a lower portion of the extension surface 6111 to provide a space in which the intake unit 62 or the fan 64 is installed, a blocking surface 6114 extending from a lower portion of the installation surface 6112 toward the inner surface of the door 50 to form a lower surface of the duct unit 61, and a limiting surface 6113 defining a lateral surface of the intake duct 611.

The extension surface 6111 may extend obliquely from the lower portion of the exhaust duct 612 and define a downward convex curve. Accordingly, the condensate flowing into the extension surface 6111 may be collected on the bottom surface of the inner case 20 as a flow rate slows down downward from the extension surface 6111.

The installation surface 6112 extends downward from the extension surface 6111 and is an area of the duct unit 61, which protrudes the most from the door 50. Accordingly, the installation surface 6112 may include the intake unit 63 and accommodate the fan 64 therein.

The installation surface 6112 may be located in parallel to a rear surface of the door 50 or the inner case 20. Thus, water flowing along the extension surface 6111 may be minimized from flowing into the installation surface 6112, thereby preventing water from flowing into the intake unit 63.

The intake unit 63 may include an intake hole 631 through which one or more of hot air and steam supplied to the inner case 20 is introduced to pass through the installation surface 6112, a fan installation unit 632 provided inside the intake hole 631 or inside the intake duct 611 to provide a space in which the fan 64 is accommodated, and a through hole 633 formed to pass through one surface of the fan installation unit 631 to guide the hot air and steam into the intake duct 611.

The fan installation unit 632 may be provided in a case shape that accommodates at least a portion of the fan 64 inside the intake duct 611. An opening of the fan installation unit 632, into which the fan 64 is inserted may correspond to the intake hole 631.

The through hole 633 may be provided in one surface of the fan installation unit 632, which faces the door 50.

The fan installation unit 632 may be provided in multiple numbers and may be arranged in a width direction of the intake duct 611.

The fan 64 may be accommodated and fixed in the fan installation unit 631.

The fan 64 may be at least partially accommodated in the fan installation unit 631. The fan 64 may be completely accommodated and fixed in the intake duct 611. The fan 64 may be provided to suck one or more of the hot air and steam supplied to the inner case 20 into the intake unit 63 and may provide power to supply one or more of the hot air and steam sucked into the intake unit 63 to the discharge unit 62.

The air moving unit 60 may include a limiting filter 66 that shields the intake hole 631 to prevent the fan 64 from being exposed to the outside or from being separated. The limiting filter 66 may be provided in a grill shape and may be coupled to an inner circumference of the intake hole 631.

The intake unit 63 may be located in parallel to the discharge unit 62. The intake unit 63 may be provided on the limiting surface 6113. However, the intake unit 63 may be excluded from being installed on the blocking surface 6114. The blocking surface 6114 may include a plurality of through holes provided in the bottom surface of the inner case 20 to prevent the intake unit 63 from interfering with the flow of hot air and steam discharged or introduced from the machine room 30.

A direction in which hot air and steam are introduced from the intake unit 63 and an extension direction of the duct unit 61 may be different from each other. Accordingly, when the hot air and steam flowing into the intake unit 63 collide directly with one surface of the duct unit 61, unnecessary flow resistance may occur, and the hot air and steam may not be fully supplied to the discharge unit 62.

To prevent this, provided may be a guide 65 that is located between the intake unit 63 and the duct unit 61 or an inner surface of the door 50 and guides at least one of the hot air and the steam introduced from the intake unit 63 to the exhaust duct 612.

The guide 65 may include one or more guide vanes 652 arranged between the through hole 633 or the fan installation unit 631 and the inner surface 52 of the door to guide air introduced from the intake unit 63 to the exhaust duct 612.

The guide vane 652 may be provided with one edge facing the intake unit 63 and the other edge facing the exhaust duct 611. The guide vane 652 may be provided convexly downward and a width thereof may correspond to 1/3 of the width of the through hole 633.

The guide vane 652 may be provided in multiple numbers and may be located at a certain distance apart from a lower portion of the through hole 633 or the intake hole 631.

The guide 65 may further include support ribs 651 on both sides of the plurality of guide vanes 652. The guide vane 652 may be provided such that a length thereof increases from a lower portion to an upper portion.

The exhaust duct 612 may extend from an upper portion of the intake duct 611 to face the pressurizer 40.

The exhaust duct 612 may be provided with a shape corresponding to the shape of the pressurizer 40.

An area of the exhaust duct 612 may be equal to or larger than an area of the pressurizer 40.

The discharge unit 62 may include an exhaust hole 621 provided to pass through an area of one surface of the exhaust duct 612, which faces at least a portion of the pressurizer 40. An area of the exhaust hole 621 may be provided to be larger than half an area of the pressurizer 40 and may be provided to be larger than the area of the intake hole 631.

Accordingly, the discharge unit 62 may supply the hot air and steam to a wider area of laundry pressurized by the pressurizer 40. The discharge unit 62 may supply hot air and steam to the laundry for a longer period of time by lowering a discharge speed of hot air and steam introduced into the exhaust duct 611.

An area of the exhaust hole 621 may be provided as an area corresponding to the pressurizer 40. The exhaust hole 621 may be provided through the entire surface of the exhaust duct 611, which faces the pressurizer 40. Thus, the discharge unit 62 may supply hot air and steam to the entire pressurizer 40.

The discharge unit 62 may further include an accommodation filter 623 provided to shield the inside of the exhaust hole 621. The accommodation filter 623 may be coupled to the inner circumference of the exhaust hole 621 and may be provided such that the support portion 41 is accommodated thereon.

The accommodation filter 623 may include a mesh member or a plurality of through holes such that one or more of hot air and steam may pass through the exhaust hole 621.

The accommodation filter 623 may be provided in a shape corresponding to the shape of the exhaust hole 621 and may prevent other components such as the support portion 41 from being introduced into the exhaust duct 612.

The accommodation filter 623 may maintain an internal cross-sectional area of the exhaust duct 612 and may also remove foreign substances discharged from the exhaust duct 612.

The accommodation filter 623 may be provided in the form of a metal mesh.

The discharge unit 62 may further include a sealer 622 that seals a space between an outer circumference of the accommodation filter 623 and an inner circumference of the exhaust hole 621. The sealer 622 may include an elastic material and may also fix the accommodation filter 623 to the exhaust hole 621.

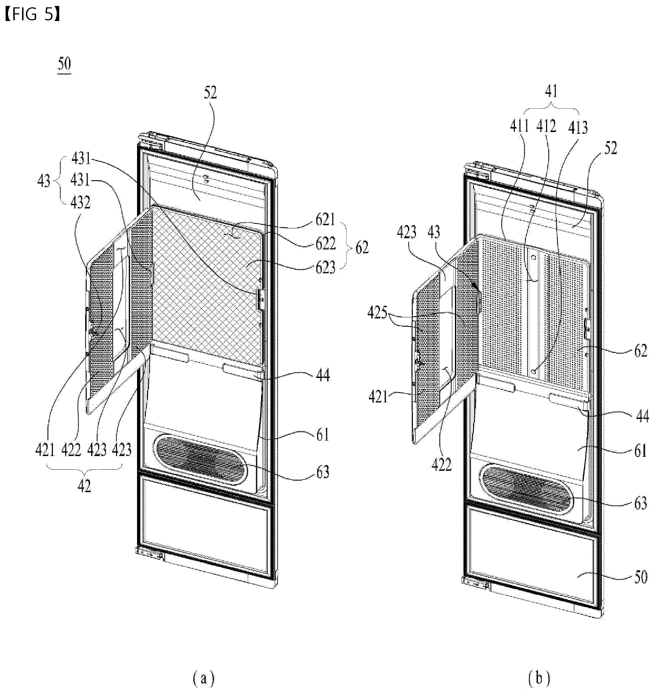

FIG. 5 illustrates a structure in which the pressurizer 40 is installed in the duct unit 61.

FIG. 5(a) illustrates a structure in which the compression portion 42 is installed in the duct unit 61 and the support portion 41 is excluded.

The duct unit 61 may be coupled and fixed to the inner surface 52 of the door, the intake unit 63 into which hot air and steam supplied to the inner case 20 are introduced may be provided at a lower portion of the duct unit 61, and the discharge unit 62 through which hot air and steam are discharged may be provided at an upper portion of the duct unit 61.

A fixing clip 44 that fixes laundry held on the holder 30 may be additionally located between the intake unit 63 and the discharge unit 62.

The discharge unit 62 may be provided with a larger area than the pressurizer 40 and may include the exhaust hole 621 that faces the pressurizer 40 to discharge hot air and steam introduced into the duct unit 61, an accommodation cover 623 provided to shield the inside of the exhaust hole 621, and the sealer 622 that seals a space between the accommodation cover 623 and the exhaust hole 621.

The accommodation cover 623 may be provided to maintain a state of being separated from the inner surface 52 of the door or the inner surface of the duct unit 61 and may be firmly fixed to the exhaust hole 621 not to be pushed toward the door 50 even when the compression portion 42 is pressurized.

The accommodation cover 623 may be provided with a porous material or mesh member to minimize flow resistance.

The pressurizer 40 may include a coupler 43 that rotatably couples the compression portion 42 to the inner surface 52 of the door or the duct unit 61.

The coupler 43 may include a hinge 431 that rotatably connects the compression portion 42 to one surface of the door 50 or the duct unit 61, and a fastening unit 433 that maintains a state in which the compression portion 42 pressurizes the support portion 41.

The coupler 43 may further include a hook 432 that is provided on the compression portion 42 and detachably connected to the fastening unit 433.

The hinge 431 may be located at one side of the duct unit 61 and the fastening unit 433 may be located at the other side of the duct unit 61.

The compression portion 42 may include a compression body 421 having a plate shape and pressurizing the laundry.

The compression portion 42 may include an internal hole 422 formed to pass through the center of the compression body 421 and the plurality of compression holes 425 formed to pass through one surface of the compression body 421.

The compression body 421 may include a first pressure body 4211 that pressurizes one side of the laundry and a second pressure body 4212 that pressurizes the other side of the laundry, and the first pressure body 4211 and the second pressure body 4212 may be spaced apart from each other by the internal hole 422.

The internal hole 422 may be provided not to pressurize the sewing line provided on the lower garment.

The compression body 421 may include avoidance grooves 423 that connect the first pressure body 4211 to the second pressure body 4212 and are located above and below the internal hole 422.

The avoidance grooves 423 may each be provided in a plate shape but may be provided curved to be spaced further from the support portion 41 than the first pressure body 4211 and the second pressure body 4212.

Even if the compression body 421 pressurizes the laundry, the avoidance grooves 423 may maintain a spaced state without coming into contact with the laundry.

The compression body 421 includes the plurality of compression holes 425 and the internal hole 422 is provided in the center of the compression body 421, and thus the hot air and steam emitted from a discharge unit 62 may pass through the compression portion 42 without change and may be recovered into the inner case 20.

FIG. 5(b) illustrates a structure in which the support portion 41 is coupled to a front side of the accommodation filter 623.

The support portion 41 may be coupled and fixed to one surface of the duct unit 61. In detail, the support portion 41 may be provided in a plate shape and may be coupled to one surface of the accommodation filter 623.

The support portion 41 may include a fixing surface 412 coupled to the accommodation filter 623, and a fixing unit 413 that couples the fixing surface 412 to at least one of the accommodation filter 623, the door 50, and the duct unit 61.

The support portion 41 may include a support body 411 that extends to both sides from the fixing surface 412 and supports one surface of the laundry.

The support body 411 may extend from the fixing surface 412 but may be spaced a certain distance from the accommodation filter 623. Accordingly, the support body 411 may be provided to be in contact with the compression body 421 and may provide elasticity to the compression body 421.

The support body 411 may be spaced apart from the accommodation filter 623, and thus the sewing line may be prevented from being pressurized against the fixing surface 412.

The support body 411 may include a first support body 4111 that extends from the fixing surface 412 to one side and is pressurized against the first pressure body 4211, and a second support body 4112 that extends from the fixing surface 412 to the other side and is pressurized against the second pressure body 4212.

The first support body 411 and the second support body 4112 may be provided symmetrically to each other.

The first support body 4111 and the second support body 4112 may include the support hole 415 through which one or more of hot air and steam may pass along a surface.

The support hole 415 may be provided to pass through the support body 411 and may transmit hot air and steam discharged from the accommodation filter 623 to laundry supported by the support body 411.

Accordingly, the hot air and steam discharged from the discharge unit 62 may pass through the laundry pressurized by the support portion 41 and the compression portion 42 without change, and the laundry may be refreshed and wrinkles may be removed therefrom even if the laundry is pressurized by the support portion 41 and the compression portion 42.

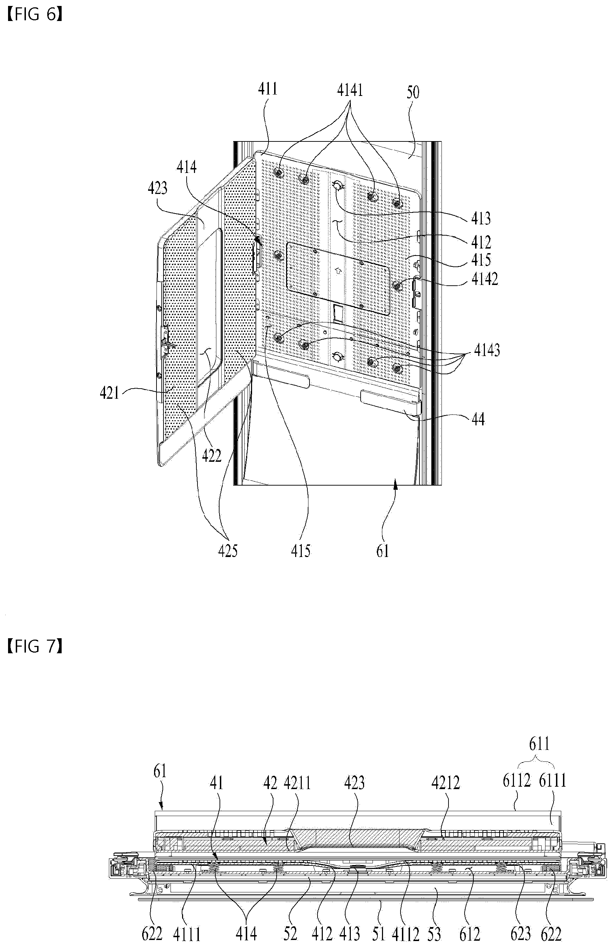

FIG. 6 illustrates the pressurizer 40 according to an embodiment.

The support portion 41 may be coupled to an inner surface of the duct unit 61, the inner surface 52 of the door, or the accommodation filter 623 through the fixing unit 413, and the first support body 4111 and the second support body 4112 may be spaced apart from the inner surface of the duct unit 61 or the inner surface 52 of the door.

As a result, even if the support portion 41 is coupled to the duct unit 61, the duct unit 61 may minimize interference with the support portion 41. Accordingly, even if the support portion 41 is pressed by the compression portion 42, a cross-sectional area of the exhaust duct 612 may be sufficiently ensured such that a flow rate of hot air and steam may be ensured.

The pressurizer 40 may further include an elastic member 414 for buffering an impact applied to the duct unit 61 during a process in which the pressurizer 40 pressurizes the support portion 41.

The elastic member 414 may include a material having elasticity and restoring force, like a spring. The elastic member 414 may have one end coupled to an inner surface of the duct unit 61 and the other end coupled to the support body 411 or the accommodation filter 623.

The elastic member 414 may be located at a certain distance apart in a width direction of the support body 411 and may also be arranged at a certain distance apart in a height direction of the support body 411.

The elastic member 414 may include at least one of a first elastic member 4141 supporting an upper portion of the support body 411, a second elastic member 4143 supporting a lower portion of the support body 411, and a third elastic member 4142 supporting a space between the upper and lower ends of the support body 411.

The first elastic member 4141 may be located symmetrically left and right with respect to the fixing surface 412, and the second elastic member 4142 may also be located symmetrically left and right with respect to the fixing surface 412. The third elastic member 4142 may also be located symmetrically left and right with respect to the fixing surface 412.

Accordingly, the pressure or impact applied to the support portion 41 may be evenly distributed to the first support body 4111 and the second support body 4112.

The first elastic member 4141 and the second elastic member 4143 may be provided symmetrically in upper and lower directions. Accordingly, the impact applied to the upper and lower portions of the support portion 41 may be evenly buffered.

The third elastic member 4143 is provided in smaller quantities than the first elastic member 4141 and the second elastic member 4142, thereby minimizing reduction in a flow path area of the duct unit 61.

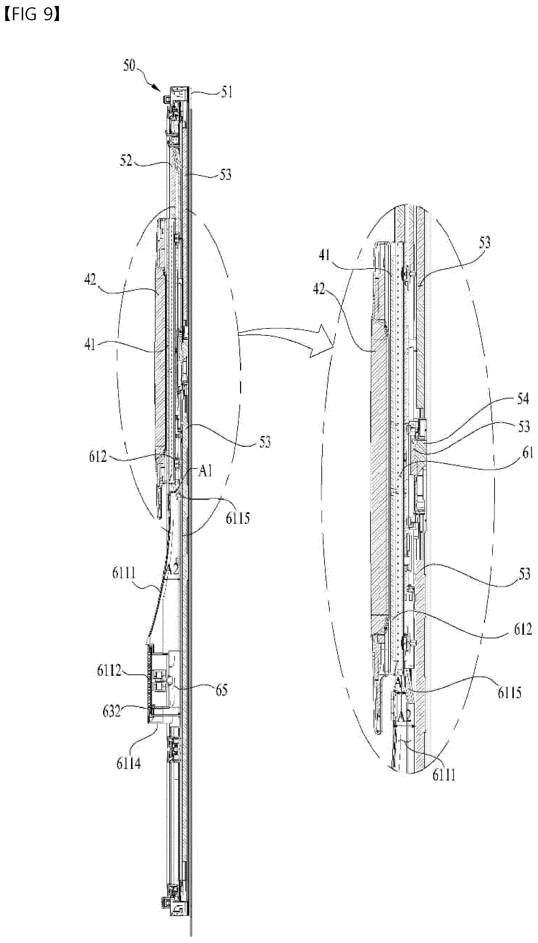

FIG. 7 is a cross-sectional view of the door 50. - Remove duct unit 61 - FIG. 9

A buffer member 53 may be provided between an outer surface 51 of the door and the inner surface 52 of the door.

The buffer member 53 may prevent heat radiated from hot air and steam flowing through the duct unit 61 from being transferred to the outside of the cabinet 10 or a control panel 54 to be coupled to the outer surface 51 of the door.

The inner surface 52 of the door may define one surface of the duct unit 61 and the elastic member 414 may be coupled and fixed thereto.

The elastic member 414 may be spaced apart along the inner surface 52 of the door or in a width direction of the duct unit 61 and may be provided symmetrically to the fixing surface 412 or the fixing unit 413.

The support portion 41 may be provided such that the first support body 4111 and the second support body 4112 symmetrically extend based on the fixing surface 412, and the first support body 4111 and the second support body 4112 may be supported by the elastic member 414 and maintained in a state of being spaced apart from the inner surface 52 of the door. Thus, an area of the duct unit 61 may be ensured and maintained to the maximum extent possible.

The elastic member 414 may indirectly support the support portion 41 while supporting the accommodation filter 623.

The compression portion 42 may be provided such that the first pressure body 4211 that pressurizes the first support body 4111 and compresses laundry and the second pressure body 4212 that pressurizes the second support body 4112 and compresses laundry are symmetrically located with respect to the avoidance grooves 423.

The laundry may be pressurized on both surfaces by having one surface in contact with the support portion 41 and the other surface pressurized against the compression portion 42. In this case, wrinkles may be removed from laundry while one or more of the hot air and steam supplied from the duct unit 61 passes through the pressurized laundry and the laundry is deodorized.

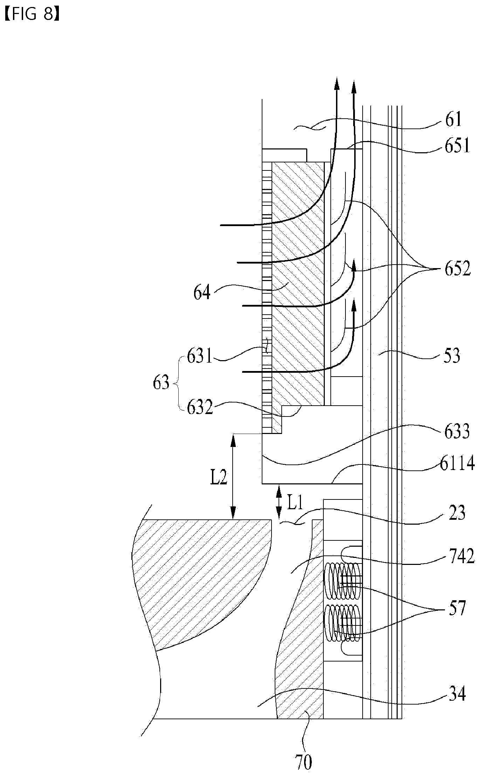

FIG. 8 illustrates a cross-sectional view of a height of the door 50.

The buffer member 53 may be provided between the outer surface 51 and the inner surface 52 of the door 50, and the air moving unit 60 may be coupled to the inner surface 52 of the door.

The air moving unit 60 includes the duct unit 61 that extends downward at a certain distance from the upper end of the door 50.

The duct unit 61 may include the exhaust duct 612 to which the support portion 41 is coupled, and the intake duct 611 that extends downward from the exhaust duct 612 and extends downward than the support portion 41 and the compression portion 42.

The control panel 54 that displays a state of the clothing processing apparatus or receives a command to operate the clothing processing apparatus may be provided between the exhaust duct 612 and the outer surface 51 of the door.

The support portion 41 and the compression portion 42 may be fixed to the exhaust duct 612, and the intake duct 611 may extend to further protrude away from the inner surface 52 of the door from the exhaust duct 612.

A surface of the intake duct 611 may be located further from the inner surface 52 of the door than a surface of the pressurizer 40 and may be located close to the rear surface of the inner case 20.

As a result, the volume of the intake duct 611 may be provided to be larger than the volume of the exhaust duct 612, and thus more hot air or steam may be sucked in, and a space for installing the fan 64 therein may be ensured.

At the same time, moisture condensed between the pressurizer 40 and the exhaust duct 612 may be induced to flow on a surface of the intake duct 611 without directly falling on the bottom surface of the inner case 20. Condensate flowing along the surface of the intake duct 611 may be easily vaporized due to the temperature of the hot air or steam supplied to the inner case 20 because a surface area of the condensate is increased.

The intake duct 611 may extend to protrude from the exhaust duct 612 such that an upper surface of the intake duct 611 faces a lower surface of the pressurizer 40 and extend such that a lower surface of the intake duct 611 faces an intake hole 23. As a result, even if the condensate is not completely vaporized on the surface of the intake duct 611, the condensate may be prevented from accumulating on the bottom surface of the inner case 20 by causing the condensate to fall into the intake hole 23.

The surface of the exhaust duct 612 or one surface facing the support portion 41 may be located in parallel to the door.

The intake duct 611 may include the extension surface 6111 that extends in a slanted or curved manner from the surface of the exhaust duct 612 and faces the lower portion of the pressurizer 40.

The extension surface 6111 may be provided to be convex downward to induce a decrease in speed when the condensate falls downward, and a lower end of the extension surface 6111 may be further away from the inner surface 52 of the door, thereby further ensuring an internal space of the intake duct 611.

The length of the extension surface 6111 may extend longer than half the length (height) of the pressurizer 40.

A flow cross-sectional area or internal spacing of the exhaust duct 612 may be provided as a first spacing A1, and a flow cross-sectional area or internal spacing of the intake duct may be provided as a second spacing A2 that is longer than the first spacing A1 due to the extension surface 6111.

The installation surface 6112 may extend downwardly below the extension surface 6111. The installation surface 6112 may be located closer to the rear surface of the inner case 20 than the pressurizer 40 and may be located further away from the door 50 than the surface of the pressurizer 40.

The intake duct 611 may include a reduced surface 6115 extending obliquely from the inner surface 52 of the door toward the pressurizer 40. The reduced surface 6115 may be provided to gradually reduce a cross-sectional area A2 of the intake duct 611 to a cross-sectional area A1 of the exhaust duct.

The reduced surface 6115 may extend upward from the inner surface 52 of the door and toward the pressurizer 40. The inner surface of the exhaust duct 612 may extend upward from the upper end of the reduced surface 6115.

Due to the reduced surface 6115, a space may be ensured between the exhaust duct 612 and the outer surface 51 of the door for installing the buffer member 53 or the control panel 54.

The reduced surface 6115 may function as a nozzle that reduces a flow area of the exhaust duct 612. Accordingly, the hot air and steam introduced into the intake duct 611 may be introduced into the exhaust duct 612 at a high speed due to the reduced surface 6115. As a result, one or more of the hot air and steam may effectively pass through the accommodation filter 623 and the pressurizer 40 even when the accommodation filter 623 and the pressurizer 40 are arranged.

The length of the installation surface 6112 may be provided to be less than the length of the extension surface 6111.

A fan installation unit 623 may be provided inside the installation surface 6112.

The fan installation unit 623 may be located between the intake hole 631 and the door 50 to provide a space in which the fan 64 is installed. The fan 64 may be provided to direct hot air and steam supplied to the inner case 20 toward the door 50.

The installation surface 6112 may be located above the machine room 70. Accordingly, the installation surface 6112 may be prevented from facing the machine room 70, and the intake hole 631 may be located above the machine room 70.

Accordingly, the intake duct 611 may be provided to sufficiently protrude from the door 50 without being restricted by the machine room 70.

The hot air and steam supplied to the inner case 20 may be completely introduced into the intake duct 611 without being obstructed by the laundry and the machine room 70.

The blocking surface 6114 may extend from the lower surface of the installation surface 6112 toward the door 50. The blocking surface 6114 may be located above the machine room 70.

FIG. 9 illustrates a positional relationship between the intake duct 611 and the machine room 70.

The door 50 may be located to form a shielding region to shield the machine room 70 below the air moving unit 60.

The shielding region may be provided as the inner surface 52 of the door and may include a buffer 57 that protrudes from the inner surface 52 of the door and is located at a front surface of the machine room 70.

The buffer 57 may be provided to prevent the door 50 and the machine room 70 from colliding with each other when the door 50 closes the opening of the cabinet 10. The buffer 57 may be provided to be in contact with the front surface of the machine room 70 and may prevent a water tank accommodated in the machine room 70 from being detached due to vibration.

The machine room 70 may be provided to communicate with the accommodation space 21 through the intake hole 23 and the discharge hole 25 provided on the bottom surface of the inner case 20.

In this case, the intake unit 63 may be provided not to face the intake hole 23 or the discharge hole 25, and thus the intake unit 63 may not impede movement of hot air and steam passing through the intake hole 23 and the discharge hole 25.

For example, when the intake hole 23 is located closer to the door 50 than the discharge hole 25, the intake unit 63 may be provided not to face the intake hole 23.

The installation surface 6112 may be provided not to face the intake hole 23. The blocking surface 6114 may be provided to at least partially overlap the intake hole 23.

The blocking surface 6114 may entirely overlap the intake hole 23 to prevent a lower endo f the installation surface 6112 from being located to overlap the intake hole 23 in a height direction. As such, interference between the air flowing into the intake hole 23 and the air flowing into the intake unit 63 may be reduced.

The intake hole 631 may be located at a certain distance from the lower end of the installation surface 6111 or the blocking surface 6114. Thus, a spacing between the intake hole 631 and the intake hole 23 may further expand to minimize interference between the air flowing into the intake hole 23 and the air flowing into the intake unit 63.

The fan installation unit 632 may also be provided at a height parallel to the intake hole 631 or may be located above the intake hole 631 rather than below the intake hole 631, thereby further expanding a spacing between the fan 64 and the intake hole 23.

The blocking surface 6114 may be located apart from the intake hole 23 by a first length L 1. This may prevent the blocking surface 6114 from directly shielding the intake hole 23.

The lower end of the intake hole 631 may be located apart from the intake hole 23 by a second length L2 that is longer than the first length L1.

The fan 64 is provided to suck air in a width direction or front-back direction of the duct unit 61. As a result, the fan 64 may be provided not to suck air in an extension direction or height direction of the duct unit 61. Accordingly, a direction of the air passing through the intake hole 23 or the discharge hole 25 or a direction of the air flowing into the fan 64 may be set differently, thereby minimizing the two airs from interfering with or disturbing each other.

The length of the blocking surface 6114 may be provided to be longer than the thickness of the fan 64. As such, a space for installing the fan 64 in the intake duct 611 may be ensured, while sufficient hot air and steam may be induced to flow into the duct unit 61.

The duct unit 61 may be located between the fan 64 and the door 50 inside the duct unit 61. The guide 65 may be provided to change the direction of air flowing from the fan 64 to the extension direction of the duct unit 61. As a result, a flow rate and volume of air flowing from the fan 64 may be maintained to the maximum and guided to the exhaust duct 612.

The guide vane 652 may be located in multiple places spaced apart from each other along the extension direction of the duct unit 61 between the fan 64 and the door 50.

The guide vane 652 may be provided to be spaced apart from the inner surface 52 of the door or the inner surface of the duct unit 61. In this way, the guide vane 652 may be prevented from interfering with flow of moving air as much as possible.

The guide vane 652 may be provided with a cross section that is convex downwards and may be provided with one end facing the fan 64 and the other end facing the exhaust duct 612.

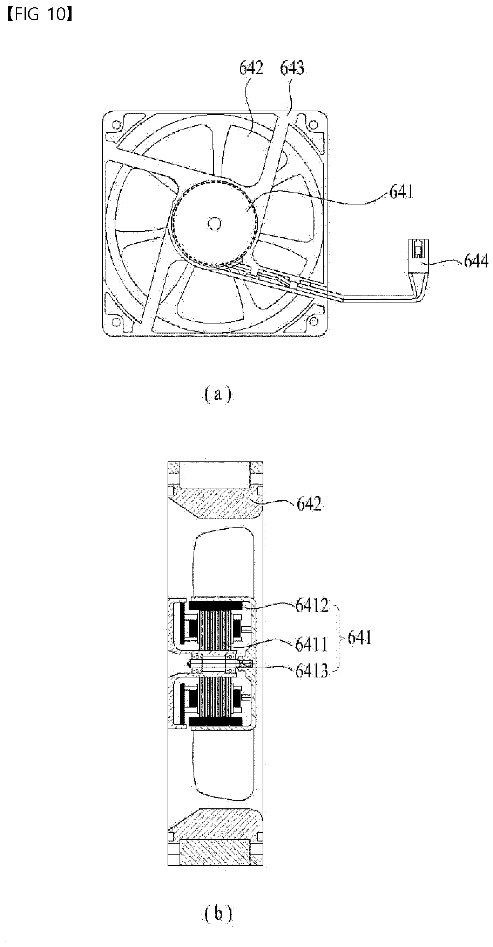

FIG. 10 illustrates an example of the fan 64.

Referring to FIG. 10(a), the fan 64 may include a driver 641 including an impeller 642 that moves air and a rotation shaft that rotates the impeller 642, and a harness 644 that supplies power to the driver 641. The fan 64 may further include a fan body 643 that rotatably accommodates the impeller 642 and is accommodated in the fan installation unit 632.

The impeller 642 may be provided with a diameter longer than the thickness of the impeller 642. That is, a diameter of the impeller 642 may be provided to be longer than an axial direction. Thus, a flow rate of at least one of the hot air and steam passing through the impeller 642 may be further increased.

The fan 64 may be accommodated and provided inside the driver 641 and the impeller 642. Accordingly, the volume occupied by the driver 641 separately from the fan body 643 or the impeller 642 may be minimized, thereby minimizing the overall volume of the fan 64.

Referring to FIG. 10(b), the driver 641 may include a stator 6411 that is fixed to the fan body 643 inside the impeller 642 and generates a rotating magnetic field, a rotor 6412 that rotates by the stator 6411, and a rotation shaft 6413 that has one end coupled to the rotor 6412 and the other end coupled to the impeller 642 and rotates together with the rotor 6412.

The rotation shaft 6413 may be located in a thickness direction of the impeller 642 and located inside the impeller 642.

The impeller 642 may be located inside the duct unit 61 such that a diameter direction is parallel to the extension direction of the duct unit 61 and a thickness direction or the direction of the rotation shaft 6413 may be located in the front-back direction. Accordingly, the length of the intake duct 611 protruding from the exhaust duct 612 may be minimized, and both the fan 64 and the guide 65 may be installed inside the intake duct 611.

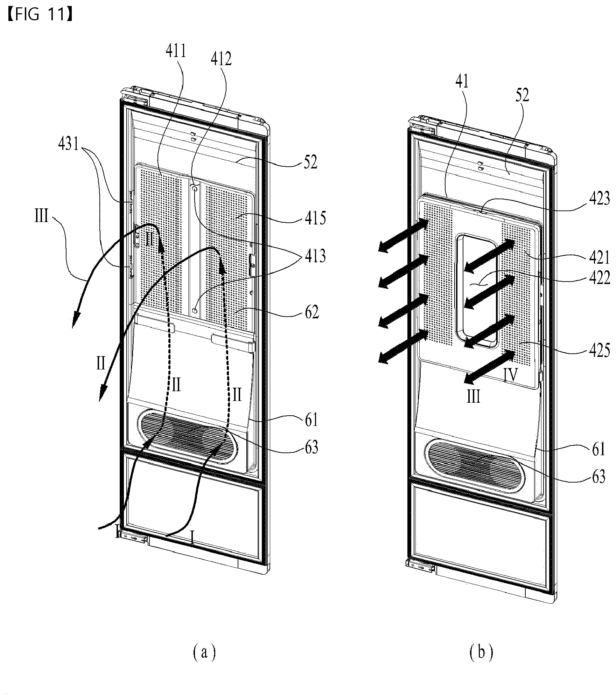

FIG. 11 illustrates a flow path of the door 50.

Referring to FIG. 11(a), the fan 64 is located closer to a lower end of the door 50 than an upper end of the door 50, and the lower end of the door 50 is located closer to the ground. Accordingly, when the fan 64 is located closer to the upper end of the door 50, vibration or noise generated from the fan 64 may be transmitted to the door 50 at a low amplitude or size.

The fan 64 may be located lower than the pressurizer 40.

When the fan 64 is driven, the air contained inside the inner case 20 is introduced toward the intake unit 63. The intake unit 63 may be provided on the installation surface 6111 parallel to the inner surface of the door 50, and thus the air may be introduced in direction I from the accommodation space 21 toward the door 50.

When one or more of hot air and steam is discharged to the inner case 20 through the discharge hole 25 in the machine room 70 or when hot air and steam are already supplied through the discharge hole 25, one or more of the hot air and steam may be introduced into the intake unit 63 by a negative pressure provided by the fan 64.

One or more of the hot air and steam may deodorize and remove wrinkles from laundry held on the hanging unit 100.

One or more of the hot air and steam introduced into the intake unit 63 passes through the intake duct 611 by power provided by the fan 64 and is introduced into the exhaust duct 612.

The intake duct 611 may be located below the exhaust duct 612, and thus the hot air and steam may move in direction II, which is a height direction.

The hot air and steam reaching the exhaust duct 612 are discharged toward the support portion 41 by the discharge unit 62.

The first support body 4111 and the second support body 4112 may each include the plurality of support holes 415, and thus one or more of the hot air and steam may pass through the support portion 41 while moving in direction III from the duct unit 61 toward the inner case 20. As a result, hot air and steam may actively reach laundry that comes into contact with or is pressurized by the support portion 41.

Referring to FIG. 11(b), the compression portion 42 may be provided to pressurize the support portion 41 to compress both surfaces of the laundry.

In this case, the support portion 41 may have an internal space between the compression portion 42 and the fixing surface 412 due to the fixing surface 412 and the compression portion 42 may have an opening due to the internal hole 422, and thus hot air and steam supplied from the support member 41 may pass through the compression portion 42 without experiencing a large flow resistance.

The compression body 41 may include the compression hole 425 corresponding to the support hole 145, and thus the hot air and steam passing through the support portion 41 may move in direction III through the compression hole 425 immediately after passing through the laundry even if the hot air and steam do not move to the internal hole 422.

As a result, air inside the inner case 20 may be introduced into the duct unit 61 by the fan 64 and may move to the pressurizer 40, and then sequentially pass through the discharge unit 63, the support hole 415, and the compression hole 425.

As a result, one or more of a sufficient amount of hot air and steam may be supplied to an area of the laundry with both surfaces pressurized by the pressurizer 40.

The fan 64 may also be controlled in reverse. That is, one or more of hot air and steam accommodated in the inner case 20 may be introduced into the discharge unit 62 and discharged to the intake unit 63.

That is, the air accommodated in the inner case 20 may move in direction IV by passing through the compression hole 425, passing through the laundry, and then flowing into the support hole 415.

Accordingly, hot air and steam may be evenly supplied to both surfaces of the laundry held on the pressurizer 40, and thus the entire laundry may be evenly deodorized or wrinkles removed therefrom. Moisture condensed in the discharge unit 62 or the pressurizer 40 may be discharged back into the inner case 20 while passing through the duct unit 61.

As a result, the laundry pressurized in the pressurizer 40 may have wrinkles removed as hot air and steam pass through the laundry, and the laundry exposed to the outside of the pressurizer 40 may have wrinkles removed as laundry is exposed to hot air and steam while being spread out by their own weight and refreshed.

Accordingly, by installing at least one of the duct unit 61, the discharge unit 62, the intake unit 63, and the fan 64 in the door 50, the pressurizer 40 may function as an iron that pressurizes laundry and provides at least one of heat and steam to remove wrinkles from laundry.

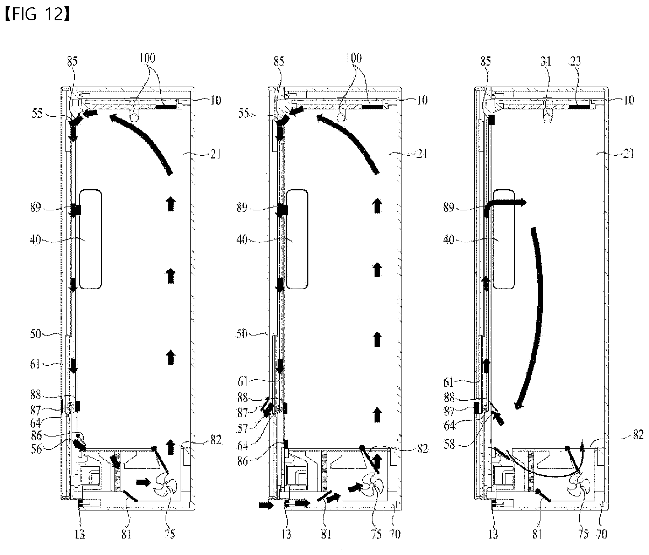

FIG. 12 illustrates a clothing processing apparatus according to another embodiment of the present disclosure.

The door 50 of the clothing processing apparatus according to the present disclosure may be provided such that air may move through the duct unit 61 regardless of the pressurizer 40.

The door 50 may include the duct unit 61 to ensure a path for air to move. The air may include one or more of hot air and steam generated in the machine room 70.

The door 50 may include the fan 64 that supplies air to the inside of the duct unit 61 or provides power to discharge air inside the duct unit 61 to the outside of the door 50.

The fan 64 may be fixedly coupled to the door 50 and may be accommodated in at least a portion of the duct unit 61. The fan 64 may be provided in the intake unit 63 that supplies air to the duct unit 61, but may also be provided in the discharge unit 62 that discharges air from the duct unit 61 to the outside.

The door 50 may further include an external discharge unit 57 that connects the duct unit 61 to the outside of the cabinet 10. Hot air or steam supplied to the door 50 may be discharged to the outside due to the external discharge unit 57.

When the duct unit 61 of the door is connected to the machine room 70 or the inner case 20, the outside of the cabinet 10 may be connected to the machine room 70 or the inner case 20 through the door 50 due to the external discharge unit 57.

The hot air supplied inside the duct unit 61 may be air that is completely dehumidified from general air. Accordingly, the dehumidified air may be discharged to the external discharge unit 57, and thus the clothing processing apparatus that dehumidifies an indoor space may function as a dehumidifier.

The inner case 20 may be connected to the outside of the cabinet 10 and the external discharge unit 57, and thus the inside of the inner case 20 may be naturally dried. Accordingly, when there is steam or condensate discharged from laundry inside the inner case 20, the condensate may be dehumidified by the external discharge unit 57.

The door 50 may include an external damper 87 that opens and closes the external discharge unit 57. The external damper 87 may electronically controlled to selectively open and close the external discharge unit 57. Accordingly, it may be possible to control whether or not the air introduced into the duct unit 61 is supplied to the external discharge unit 57.

The clothing processing apparatus according to the present disclosure may include a communication hole 13 for introducing outside air into the machine room 70. The communication hole 13 may be provided in the door 50, but may be directly connected to the machine room 70 exposed at the lower portion of the door 50.

The clothing processing apparatus according to the present disclosure may include a mechanical damper 81 that selectively connects the communication hole 13 to the machine room 70. The mechanical damper 81 may be provided to selectively open and close the communication hole 13 and may be provided to selectively connect the communication hole 13 and the circulation duct 74.

When the mechanical damper 81 connects the communication hole 13 to the circulation duct 74, outside air may be introduced into the machine room 70 to be dehumidified, and may be resupplied to the inside of the inner case 20 or the outside of the door 50.

When the mechanical damper 81 blocks communication between the communication hole 13 and the circulation duct 74, only the air of the inner case 20 or the duct unit 61 may circulate in the machine room 70.

The door 50 may further include a machine guide 56 that directly connects the duct unit 61 to the machine room 70. The machine guide 56 may be provided in a duct shape extending from the duct unit 61 toward the machine room 700.

The machine guide 56 may be provided to extend from the duct unit 61 toward the intake hole 23 or the discharge hole 25 or may be provided to extend from the duct unit 61 toward the intake hole 23 or the discharge hole 25, but may be located to be spaced apart from the intake hole 23 or the discharge hole 25.

The door 50 may further include a guide damper 86 that opens and closes the machine guide 56. Accordingly, when the guide damper 86 is opened, the air in the duct unit 61 may be guided directly to the machine room 70 or air may be supplied directly from the machine room 70 to the duct unit 61.

The door 50 may further include an inlet 55 that connects the duct unit 61 to the inner case 20 or the inside of the cabinet 10. The inlet 55 may be provided separately from the intake unit 63.

The door 50 may further include an inlet damper 85 that selectively opens and closes the inlet 55.

The door 50 may include at least one of an intake damper 88 that selectively opens and closes the intake unit 63 and an exhaust damper 89 that selectively opens and closes the exhaust unit 63.

The machine room 70 may further include a case damper 82 that selectively opens and closes a discharge hole 25 communicating with the inner case 20.

The clothing processing apparatus according to the present disclosure may perform various functions by controlling one or more dampers 80 that selectively connects the duct unit 61 provided in the door 50 to the fan 64 installed in the duct unit 61, the duct unit 61, or the machine room 70.

Referring to FIG. 12(a), the case damper 82, the inlet damper 85, and the guide damper 86 may be controlled to be open, and the mechanical damper 81, the external damper 87, the intake damper 88, and the exhaust damper 89 may be controlled to be closed.

When the machine room 70 and the fan 64 are controlled to operate, air inside the inner case 20 may flow into the duct unit 61 and into the machine room 70. The air introduced into the machine room 70 may be dehumidified in the machine room 70 and supplied again to the inner case 20.

When this process is repeated, the inside of the inner case 20 and the inside of the duct unit 61 may be dehumidified, thereby preventing growth of bacteria, and the like. At the same time, when the laundry is held on the inner case 20, the laundry may also be deodorized or wrinkles removed therefrom.

Referring to FIG. 12(b), the case damper 82, the mechanical damper 81, the inlet damper 85, and the external damper 88 may be controlled to be opened, and the intake damper 88 and the exhaust damper 89 may be controlled to be closed.

In this case, when the fan 64 and the machine room 70 are provide to operate, the outside air may be introduced into the circulation duct 64, dehumidified, heated, and introduced into the inner case 20 and the duct unit 61. Thereafter, the air introduced into the duct unit 61 may be discharged outside the cabinet 10 through the external discharge unit 57.

In this process, the inner case 20 and the duct unit 61 are dehumidified, thereby preventing growth of bacteria, and the like. At the same time, the outside air of the cabinet 10 is dehumidified in the machine room 70 and discharged back to the outside of the cabinet 10, and thus surrounding air in which the cabinet 10 is located may be dehumidified.

When laundry is accommodated inside the inner case 20, the laundry may be dehumidified while being deodorized, and the deodorized air may be discharged outside the cabinet 10.

Referring to FIG. 12(c), only the intake damper 88, the exhaust damper 89, and the case damper 82 may be controlled to be opened, and all dampers may be controlled to be closed.

When the machine room 70 and the fan 64 are driven, the hot air supplied to the machine room 70 is supplied to the inner case 20 and then flows into the duct unit 61.

Air introduced into the duct unit 61 may be discharged to the discharge unit 62.

Accordingly, when laundry is held on the inner case 20, the laundry may be refreshed by the air supplied from the machine room 70 and the air discharged from the duct unit 61.

In this case, the pressurizer 40 may not be installed in the door 50. In this case, laundry held on the holder 100 may be exposed to hot air supplied from the duct unit 61 of the door 50 and hot air supplied from the machine room 70 at a lower portion. In this case, depending on a method of controlling the fan 64 and the machine room 70, the hot air may be supplied only from the discharge unit 62 of the duct unit 61, only from the machine room 70, or from both the discharge unit 62 and the discharge hole 25.

This allows the surface of the laundry may be evenly exposed to hot air and steam. Depending on a degree of contamination and wrinkling, specific areas of laundry may be exposed to one or more of hot air and steam.

Even if the pressurizer 40 is installed in the door 50, the same effect as above may be derived when the laundry is not pressurized by the pressurizer 40. That is, the hot air passing through the discharge unit 62 may pass through the pressurizer 40 without change and may be supplied to laundry held on the holder 100 or to an inner surface of the inner case 20.

When the pressurizer 40 is installed in the door 50 and laundry is pressurized in the pressurizer 40, one or more of the hot air and steam discharged from the discharge unit 62 may intensively refresh laundry pressurized in the pressurizer 40, and one or more of hot air and steam discharged from the machine room 70 may intensively refresh laundry held on the holder 100.

One or more of hot air and steam discharged from the machine room 70 may can refresh laundry exposed to the outside of the pressurizer 40 during a process of refreshing laundry held on the holder 100 or the inside of the inner case 20, and may also refresh laundry that is introduced into the intake unit 63 and pressed against the pressurizer 40.

Air discharged from the duct unit 61 or the machine room 70 into the inner case 20 may be introduced into the intake hole 23 and dehumidified and heated.

The present disclosure may be implemented in various modified forms, but the scope of the present disclosure is not limited to the embodiments. Therefore, when a modified embodiment includes a component of the claims of the present disclosure, it should be considered to fall within the scope of the present disclosure.

## Claims

1. A clothing processing apparatus comprising:
a cabinet having an opening at a front side;
an inner case provided inside the cabinet to accommodate laundry;
a machine room provided at a lower portion of the inner case and configured to generate hot air or steam supplied to an inside of the inner case;
a door rotatably coupled to the cabinet to open and close the opening and on which the laundry is to be held;
a pressurizer rotatably coupled to the door and configured to pressurize laundry held on the door; and
an air moving unit provided in the door and configured to supply at least one of the hot air or the steam to the pressurizer,
wherein the air moving unit includes:
a duct unit provided in the door and configured to move at least one of the hot air or the steam in one direction; and
an exhaust unit formed to pass through the duct unit and configured to supply at least one of the hot air or the steam to the pressurizer.

2. The clothing processing apparatus of claim 1, wherein the exhaust unit is located to at least partially overlap the pressurizer.

3. The clothing processing apparatus of claim 2, wherein the pressurizer includes:
a support facing the duct unit or the exhaust unit and configured to support one surface of the laundry; and
a compressor rotatably coupled to the door or the support and configured to pressurize the laundry toward the support, and
at least one of the support or the compressor includes a plurality of holes through which one or more of the hot air or the steam discharged from the exhaust unit passes.

4. The clothing processing apparatus of claim 3, wherein an area of the exhaust unit is larger than half an area of the support.

5. The clothing processing apparatus of claim 3, wherein the exhaust unit further includes:
an exhaust hole through which the hot air or the steam is discharged to pass through the duct unit; and
an accommodation cover configured to shield the exhaust hoe and facing the support, and
the accommodation cover is configured to guide the hot air or the steam discharged from the exhaust hole to the guide.

6. The clothing processing apparatus of claim 3, wherein the air moving unit further includes an intake unit into which at least one of the hot air and the steam supplied to the inner case is introduced to pass through the duct unit.

7. The clothing processing apparatus of claim 6, wherein the intake unit has a less area than an area of the exhaust unit.

8. The clothing processing apparatus of claim 6, wherein the intake unit is spaced apart from the pressurizer.

9. The clothing processing apparatus of claim 6, wherein the intake unit is located below the exhaust unit.

10. The clothing processing apparatus of claim 6, wherein the intake unit is located closer to a lower end of the door or a lower end of the duct unit than to an upper end of the door or an upper end of the duct unit.

11. The clothing processing apparatus of claim 10, further comprising:
a hanging unit provided in an upper portion of the inner case and on which the laundry is held; and
a holder located above the pressurizer in an inner surface of the door and on which the laundry is held, and
wherein the intake unit is provided not to face the hanging unit or the holder.

12. The clothing processing apparatus of claim 6, wherein a through hole communicating with the machine room is provided in a bottom surface of the inner case, and
the intake unit is located not to face the through hole.

13. The clothing processing apparatus of claim 6, wherein the intake unit is located in parallel to the exhaust unit.

14. The clothing processing apparatus of claim 6, wherein the duct unit further includes:
an exhaust duct facing the pressurizer and having the exhaust unit; and
an intake duct extending downward from the exhaust duct and including the intake unit.

15. The clothing processing apparatus of claim 14, wherein the intake duct protrudes further toward an inside of the inner case than the exhaust duct.

16. The clothing processing apparatus of claim 15, wherein the intake duct is located above the machine room.

17. The clothing processing apparatus of claim 15, wherein the air moving unit further includes a fan provided inside the intake duct and configured to suck one or more of the hot air and the steam supplied to the inner case into the intake unit.

18. The clothing processing apparatus of claim 17, wherein the intake duct includes:
an extension surface extending from the exhaust duct toward an inside of the inner case; and
an installation surface provided to face a rear surface of the inner case below the extension surface and providing a space in which the intake unit or the fan is installed.

19. The clothing processing apparatus of claim 18, wherein the intake duct further includes a blocking surface that extends from a lower portion of the installation surface to face a bottom surface of the inner case and blocks the intake unit or the fan from being exposed to a through hole provided in the bottom surface of the inner case.

20. The clothing processing apparatus of claim 17, wherein the fan includes:
an impeller configured to move the hot air or the steam to the exhaust duct from an inside of the intake duct; and
a driver including a rotation shaft that rotates the impeller, and
a direction of the rotation shaft is located in parallel to a thickness direction of the door.

21. The clothing processing apparatus of claim 17, wherein the air moving unit includes a guide located between the intake unit and the door and configured to guide at least one of the hot air and the steam introduced from the intake unit to the exhaust duct, and
the guide includes one or more vanes located between the fan and the door.

22. A clothing processing apparatus comprising:
a cabinet having an opening at a front side;
an inner case provided inside the cabinet to accommodate laundry;
a machine room provided at a lower portion of the inner case and configured to generate air or steam supplied to an inside of the inner case; and
a door coupled to the cabinet and configured to open and close the opening,
wherein the door includes:
a duct unit providing a space in which at least one of the air and the steam move in one direction; and
a fan configured to move at least one of the air or the steam from the duct unit.

23. The clothing processing apparatus of claim 22, wherein at least a portion of the fan is installed inside the duct unit.

24. The clothing processing apparatus of claim 22, wherein the duct unit includes an inlet into which at least one of air inside the inner case or hot air and steam supplied from the machine room is introduced.

25. The clothing processing apparatus of claim 24, wherein the door includes:
an external discharge unit configured to guide at least one of the air, the hot air, or the steam introduced into the inlet to an outside of the cabinet; and
an external damper configured to open and close the external discharge unit.

26. The clothing processing apparatus of claim 24, wherein the door includes an internal circulation unit configured to re-guide at least one of the air, the hot air, or the steam introduced into the inlet to the machine room, and an internal damper configured to open and close the internal circulation unit.

27. A clothing processing apparatus comprising:
a cabinet having an opening at a front side;
an inner case provided inside the cabinet to have an accommodate space in which laundry is accommodated;
a machine room provided at a lower portion of the inner case and configured to generate hot air or steam supplied to an inside of the inner case; and
a door rotatably provided in the cabinet and configured to open and close the opening to hold the laundry,
wherein the door includes:
a duct unit providing a path through which at least one of the hot air and the steam moves;
an intake unit into which at least one of the hot air or the steam is introduced to pass through the duct unit; and
an exhaust unit spaced apart from the intake unit and configured to discharge at least one of the hot air or the steam introduced into the intake unit toward the laundry.

28. The clothing processing apparatus of claim 27, wherein the duct unit is located in a length direction of the door and is located above the machine room.

29. The clothing processing apparatus of claim 27, wherein the exhaust unit is provided to pass through one surface of the duct unit, which faces an inside of the inner case.

30. The clothing processing apparatus of claim 27, wherein the intake unit is provided to pass through one surface of the duct unit, which faces an inside of the inner case.

31. The clothing processing apparatus of claim 27, further comprising a fan provided inside the duct unit and configured to move one or more of the hot air and the steam to the exhaust unit from the intake unit.
